# FASCICULE DE BREVET EUROPEEN

(11) **EP 3 618 915 B1**
(45) Date de publication et mention de la délivrance du brevet: **24.04.2024**
(21) Numéro de dépôt: 18735350.3
(22) Date de dépôt: 02.05.2018
(51) Int. Cl.: A61M 37/00

(54) **SYSTEME DE BRIDAGE ET DE GUIDAGE D'UN FAISCEAU D'AIGUILLES SUR UN TUBE DE TATOUAGE**
SYSTEM ZUM KLEMMEN UND FÜHREN EINES NADELBÜNDELS AUF EINEM TÄTOWIERROHR
SYSTEM FOR CLAMPING AND GUIDING A NEEDLE BUNDLE ON A TATTOO TUBE

(30) Priorité: 03.05.2017 FR 1753865
(43) Date de publication de la demande: 11.03.2020
(73) Titulaire: I T C, 54850 Messein (FR)
(72) Inventeur: GUIGON, Pascal, 54330 Vitrey (FR); DAMERON, Philippe, 54112 Allamps (FR); PACAUD, Christophe, 55140 Champougny (FR)
(74) Mandataire: RVDB
(86) Numéro de dépôt international: PCT/FR2018/051082
(87) Numéro de publication internationale: WO 2018/202989

(56) Documents cités:
- US-A1- 2010 036 317
- US-A1- 2012 192 681
- US-A1- 2012 265 232
- US-A1- 2015 151 098
- US-A1- 2016 038 176

## Description

### Domaine technique

La présente invention se rapporte au domaine du tatouage et concerne tout particulièrement le bridage et le guidage d'un faisceau d'aiguilles sur un tube de tatouage, ledit tube étant monté sur une machine de tatouage, ledit faisceau étant entraîné en translation par un dispositif d'actionnement de la machine en vue de piquer la peau et de réaliser une pigmentation de celle-ci avec de l'encre.

### Etat de la technique

Les tatouages sont pratiqués depuis des millénaires partout dans le monde pour diverses raisons (symbole, coutume, religion, esthétisme, appartenance à un groupe ...). Un tatouage consiste en un dessin ou un symbole réalisé en injectant de l'encre dans la peau, entre le derme et l'épiderme, au moyen d'un faisceau d'aiguilles qui peut comporter une ou plusieurs aiguilles selon le tatouage à réaliser et les étapes de réalisation de celui-ci.

Le faisceau d'aiguilles (tige y compris) mesure généralement de l'ordre de cent quarante millimètres de longueur (140 mm) et comporte dans sa partie avant une ou plusieurs aiguilles qui servent à piquer la peau pour faire rentrer l'encre sous l'épiderme. Le faisceau comporte également une tige à laquelle la ou les aiguilles sont assujetties. Le faisceau d'aiguilles est monté dans un tube de tatouage qui comprend un corps de maintien et un module d'extrémité par lequel sortent les pointes du faisceau d'aiguilles. L'introduction d'une encre dans le module s'effectue en activant le faisceau d'aiguilles et en trempant l'extrémité dudit module dans un contenant d'encre, le mouvement de va-et-vient dudit faisceau d'aiguilles dont les extrémités sortent de l'extrémité du module permet à l'encre de remonter dans l'extrémité dudit module et de constituer une réserve d'encre permettant au tatoueur d'exécuter le tatouage en rechargeant régulièrement cette réserve chaque fois qu'elle se vide. Le module comprend une ouverture dans sa partie avant, au-dessus de son extrémité, cette ouverture limitant la réserve d'encre et permettant de visualiser la quantité d'encre restant dans ledit module. Cette ouverture permet également d'avoir une pression dans l'extrémité du module égale à celle du milieu ambiant, ce qui facilite l'introduction de l'encre dans ladite extrémité. Le tube permet également la manipulation du faisceau d'aiguilles durant l'opération de tatouage, le tatoueur maniant le corps de maintien.

Durant cette opération de tatouage, le faisceau d'aiguilles est activé en va-et-vient selon son sens longitudinal de sorte que les pointes des aiguilles, couvertes d'encre, sortent légèrement de l'extrémité distale du module pour piquer la peau et transférer l'encre sous l'épiderme, puis remontent dans le module pour baigner dans l'encre et s'en recouvrir avant de redescendre.

L'opération de tatouage est réalisée au moyen d'une machine de tatouage qui comprend un corps et un dispositif d'actionnement. Le tube est fixé au corps, des moyens de réglage de la position du tube sur le corps permettant d'ajuster la longueur des pointes des aiguilles qui sort de l'extrémité avant du module pour pénétrer plus ou moins la peau. Le dispositif d'actionnement agit sur l'extrémité arrière de la tige du faisceau d'aiguilles pour activer sa descente dans le module. La remontée du faisceau d'aiguilles dans le module est réalisée soit par le dispositif d'actionnement, lorsque l'extrémité arrière de la tige est fixée au dispositif d'actionnement, soit par un organe de rappel agencé entre le faisceau d'aiguilles et le module, voire entre ledit faisceau et le corps de maintien.

Durant le fonctionnement de la machine de tatouage, le faisceau d'aiguilles se déplace en translation à une fréquence variant entre quatre-vingts et cent-cinquante coups par seconde (80 à 150 piqûres de la peau par seconde). Il est donc très important de prévoir un système de bridage du faisceau d'aiguilles pour le bon fonctionnement de la machine de tatouage. En effet, seul le système de bridage permet de minimiser les vibrations au niveau des pointes des aiguilles et d'assurer un guidage correct qui permettra à l'encre de s'écouler sans trop de projections et au tatoueur de pouvoir tracer des traits droits et précis.

Traditionnellement, ce système de bridage est mis en oeuvre au moyen d'un lien élastique agencé entre le corps de la machine de tatouage et la partie arrière de la tige du faisceau d'aiguilles qui dépasse de l'extrémité arrière du tube, ce qui permet d'exercer une tension sur ladite partie arrière. L'efficacité de cette mise en oeuvre reste cependant médiocre. En effet, trop de tension fait peiner le moteur du dispositif d'actionnement et cintre le faisceau d'aiguilles qui risque de frotter contre le tube. Au contraire, le manque de tension provoque un mauvais guidage du faisceau d'aiguilles au niveau des pointes et des perturbations de l'écoulement de l'encre.

Les systèmes de bridage et de guidage décrits dans la demande de brevet US2012192681A1 visent à pallier ces inconvénients. Ces systèmes de bridage et de guidage sont configurés pour être rapportés sur le pourtour extérieur du module du tube, une pièce de bridage descendant dans l'ouverture du module ou au niveau du bord périphérique de celle-ci pour prendre appui contre la partie avant de la tige du faisceau d'aiguilles et guider ledit faisceau en translation. La position de la pièce de bridage dans la partie avant, sur le module, à proximité des pointes des aiguilles, minimise les vibrations et assure un guidage beaucoup plus précis qu'en disposant le bridage dans la partie arrière de la tige dudit faisceau. Plusieurs inconvénients majeurs demeurent malgré tout avec ces systèmes de bridage et de guidage décrits dans US201292681A1.

En effet, durant l'opération de tatouage, le tatoueur qui manipule le tube peut mettre ses doigts en contact avec le système de bridage et de guidage et malencontreusement déplacer la pièce de bridage qui ne remplit alors plus convenablement sa fonction, voire plus du tout.

En outre, le système de bridage et de guidage n'est pas universel et dépend de la forme de l'ouverture sur le module. Un tel système de bridage et de guidage ne peut donc pas être adapté sur tous les modules sans ajustement de celui-ci au modèle de module utilisé par le tatoueur.

Par ailleurs, le faisceau d'aiguilles est fixé à la tige par soudure puis l'ensemble est stérilisé pour des questions d'hygiène. Cette soudure provoque des bavures dans la zone d'assemblage du faisceau d'aiguilles avec la tige. Lors de l'activation du faisceau d'aiguilles, le système de bridage et de guidage vient prendre appui dans ladite zone de soudure. La présence des bavures empêche le guidage convenable de la tige et créé des à-coups du faisceau d'aiguilles en fonctionnement. Pour pallier ce problème, les tatoueurs liment les bavures, ce qui a pour conséquence néfaste de rendre le faisceau d'aiguilles non stérile au moment de son utilisation.

Il est également connu les systèmes décrits dans les demandes de brevet US2012265232A1, US2015151098A1 et US2010036317A1.

### Résumé de l'invention

L'invention est définie dans la revendication 1. D'autres aspects de l'invention sont présentés dans les revendications 2 à 15.

La présente invention a pour objectif de pallier les inconvénients précités. A cet effet, l'invention concerne un système de bridage et de guidage d'un faisceau d'aiguilles de tatouage qui comprend un corps creux, de préférence tubulaire, comprenant un côté avant et un côté arrière. Ce corps est configuré pour être emboîté dans une position définie à l'intérieur d'un module d'un tube de tatouage et pour recevoir une partie du faisceau d'aiguilles, par exemple une tige ou un coulisseau dudit faisceau. L'avant et l'arrière sont définis par le sens du faisceau d'aiguilles et du tube de tatouage, les pointes des aiguilles définissant le côté avant.

En outre, le système de bridage et de guidage comprend un bras qui s'étend vers l'avant au-delà du côté avant du corps auquel il est fixé. Le bras comprend une extrémité recourbée munie d'une encoche configurée pour recevoir ladite partie du faisceau d'aiguilles. Par ailleurs, ce bras est configuré pour que l'encoche appuie sur ladite partie du faisceau d'aiguilles, par exemple ladite tige ou ledit coulisseau.

Le logement du bras et du corps à l'intérieur du tube de tatouage évite tout contact avec le système de bridage et de guidage durant la manipulation dudit tube et la réalisation du tatouage. L'extension du bras vers l'avant permet, en outre, de rapprocher vers l'avant l'encoche en appui sur la partie du faisceau d'aiguilles, de manière suffisamment proche pour assurer un guidage convenable dudit faisceau d'aiguilles sans vibration.

Dans une réalisation du système de bridage et de guidage objet de l'invention, le corps est configuré pour être emboîté à l'intérieur d'un corps de maintien du tube de tatouage. Cette conception permet de disposer d'un système de bridage et de guidage complètement indépendant du module et du corps de maintien, ce qui permettra de remplacer uniquement le module et le système de bridage et de guidage sur le tube de tatouage après usage pour conserver et réutiliser uniquement le corps de maintien. On peut toutefois envisager des variantes de réalisation. A ce titre, selon une variante, le système de bridage et de guidage comprend un corps qui constitue un prolongement avant d'un corps de maintien du tube de tatouage auquel il est intégré, dans quel cas l'ensemble du tube de tatouage sera jeté après usage. On pourrait également prévoir un pré-assemblage entre le système de bridage et de guidage et le module, ledit ensemble étant assemblé sur le corps de maintien au moment de la réalisation du tatouage, cette variante permettant de ne jeter que le module et le système de bridage et de guidage après usage.

Selon une réalisation du système de bridage et de guidage objet de l'invention, le corps comprend un axe de révolution, l'encoche étant positionnée sensiblement dans ledit axe pour prendre appui sur une tige du faisceau d'aiguille. Cela permet de guider le faisceau d'aiguilles en restant dans l'axe du tube de tatouage qui se confond avec l'axe de révolution du corps lors de l'emboîtement dudit corps dans le module. Dans une réalisation préférentielle dudit système selon l'invention, le bras est incliné par rapport à l'axe de révolution pour que son extrémité recourbée soit rapprochée dudit axe, ce qui permet de minimiser la longueur de cette extrémité recourbée munie de l'encoche. Cela permet également d'éviter au bras de buter contre l'intérieur du module, du fait de la flexibilité dudit bras.

Selon une autre réalisation du système de bridage et de guidage objet de l'invention, le corps comprend un trou configuré pour guider en translation un coulisseau du faisceau d'aiguilles. Ce coulisseau est assujetti à une tige qui reçoit une ou plusieurs aiguilles, le tout formant ledit faisceau d'aiguilles. Cette mise en oeuvre correspond à une réalisation où le module, le système de bridage et le faisceau d'aiguilles sont préassemblés et forment un ensemble qui est monté sur le corps de maintien du tube au moment de la réalisation du tatouage. Selon cette réalisation, de préférence, le bras est configuré pour que son encoche prenne appui sur une partie avant du coulisseau. Selon cette réalisation, de préférence, le corps comprend une extension qui prolonge vers l'avant ledit corps, l'extension comprenant un plot pour le maintien d'un élastique de rappel du faisceau d'aiguilles. De préférence, cette extension comprend une fente dans laquelle est logé le bras. On pourrait toutefois prévoir une variante où le bras est intégré à cette extension et la prolonge vers l'avant.

Selon le système de bridage et de guidage objet de l'invention, le bras est flexible vis-à-vis du corps. On choisira pour cela une matière assurant une flexibilité convenable. Cette flexibilité du bras qui s'étend vers l'avant dans le module permet d'absorber les vibrations et de garantir un appui convenable de l'encoche contre la tige du faisceau d'aiguilles. Par ailleurs, pour une réalisation particulière où le bras disposerait d'une longueur importante dimensionnée pour que son encoche prenne appui au plus proche de l'extrémité de la tige, à proximité de la soudure permettant de fixer le faisceau d'aiguilles prolongeant ladite tige, cette flexibilité du bras permettrait de s'affranchir de la présence éventuelle de résidus ou bavures de soudure. La flexibilité du bras permettrait en effet à l'encoche de se dégager des bavures pour conserver un mouvement de va-et-vient du faisceau d'aiguilles, sans saccade. On conserve ainsi un faisceau d'aiguille stérile puisque le tatoueur n'est pas incité à limer les bavures. Toutefois, de préférence, le bras sera d'une longueur plus petite, dimensionnée pour que son encoche prenne appui sur une partie de la tige dégagée de la soudure afin d'éviter tout contact avec celle-ci. En évitant tout contact de l'encoche sur la soudure, on supprime tout risque de présence de copeaux de matière et d'usure de l'encoche dues au frottement entre lesdits éléments durant la translation en va-et-vient du faisceau d'aiguilles.

Selon le système de bridage et de guidage objet de l'invention, le corps comprend un élément de butée configuré pour arrêter la position dudit système dans le sens de la longueur du tube de tatouage lors de l'emboîtement du corps dans le module. Cela permet de définir précisément la position de l'extrémité recourbée du bras dans le tube de tatouage et, ainsi, la position d'appui de l'encoche sur la tige du faisceau d'aiguilles.

Selon le système de bridage et de guidage objet de l'invention, le corps comprend un élément de blocage configuré pour bloquer la rotation dudit système vis-à-vis du module du tube de tatouage dans lequel il est emboîté. Cela permet de définir précisément le sens de l'appui exercé par le bras sur la tige du faisceau d'aiguilles afin que l'effort d'appui soit en opposition avec l'effort exercé par le tatoueur appuyant avec la ou les aiguilles sur la peau.

L'invention concerne également un tube de tatouage qui comprend un module, un corps de maintien et un système de bridage et de guidage présentant les caractéristiques précitées. Le corps du système de bridage et de guidage est emboîté dans une partie arrière du module.

Le module est monté de manière amovible vis-à-vis du corps de maintien et du système de bridage et de guidage, ce qui permet son remplacement après usage. Le corps de maintien et le système de bridage et de guidage peuvent être assemblés de manière amovible, selon une première réalisation, ou constitués d'une seule et même pièce, selon une seconde réalisation.

Le module comprend à son extrémité avant un embout. Le module comprend également un contour muni d'une ouverture agencée à proximité de l'embout. Le système de bridage et de guidage a son encoche positionnée en amont de l'ouverture lors de son emboîtement sur le module. Cela permet d'éviter le contact d'un doigt du tatoueur sur l'extrémité recourbée du bras, en pénétrant ledit doigt dans l'ouverture par inadvertance.

Dans une réalisation du tube de tatouage, la partie arrière du module comprend un élément de blocage recevant un élément de blocage complémentaire sur le système de bridage et de guidage lors de l'emboîtement du corps dudit système dans ladite partie arrière dudit module, de sorte à bloquer la rotation entre lesdits éléments. Cela permet de définir précisément la direction d'appui de l'encoche sur la tige du faisceau d'aiguilles, à l'intérieur du module, notamment vis-à-vis de la position de l'ouverture sur ce module.

Selon une réalisation du tube de tatouage, celui-ci comprend des moyens de préhension facilitant sa manipulation. Ces moyens de préhension peuvent être mis en oeuvre directement sur le corps de maintien. Ces moyens de préhension peuvent également comporter un manchon s'adaptant sur le corps de maintien.

### Brève description des figures

Les caractéristiques et avantages de l'invention apparaîtront à la lecture de la description suivante s'appuyant sur des figures, parmi lesquelles :
- La figure 1 illustre une vue d'ensemble d'un premier mode de réalisation d'un tube de tatouage équipé d'un système de bridage et de guidage ;
- La figure 2 illustre le module et le système de bridage et de blocage sur le tube de tatouage de la figure 1 ;
- La figure 3 illustre le module des figures 1 et 2 ;
- La figure 4 illustre le système de bridage et de guidage des figures 1 et 2,
- Les figures 5 et 6 illustrent respectivement deux pièces constituant un corps de maintien sur le tube de tatouage de la figure 1 ;
- La figure 7 illustre un faisceau d'aiguilles de tatouage ;
- Les figure 8 et 9 illustrent deux vues éclatées d'une variante de tube de tatouage s'apparentant au tube de tatouage de la figure 1, seul changeant légèrement la conception du corps de maintien en deux pièces ;
- La figure 10 illustre une autre vue du système de bridage et de guidage de la figure 4 ;
- La figure 11 illustre une autre variante du tube de tatouage ;
- La figure 12 est une vue en coupe longitudinale du tube de tatouage de la figure 11 ;
- La figure 13 illustre le corps de maintien et le système de bridage et de guidage mis en oeuvre sur le tube de tatouage de la figure 11, lesdits éléments étant constitués d'une seule et même pièce ;
- La figure 14 illustre un manchon mis en oeuvre sur le tube de tatouage de la figure 11 ;
- La figure 15 illustre une variante de module mis en oeuvre sur le tube de tatouage de la figure 11.
- Les figures 16 à 21 illustrent une autre variante de mise en oeuvre du système de bridage conçu pour

### Description détaillée

Dans la suite de la description, les mêmes références sont utilisées pour décrire les mêmes caractéristiques ou leurs équivalents selon les différentes variantes de réalisation du tube de tatouage.

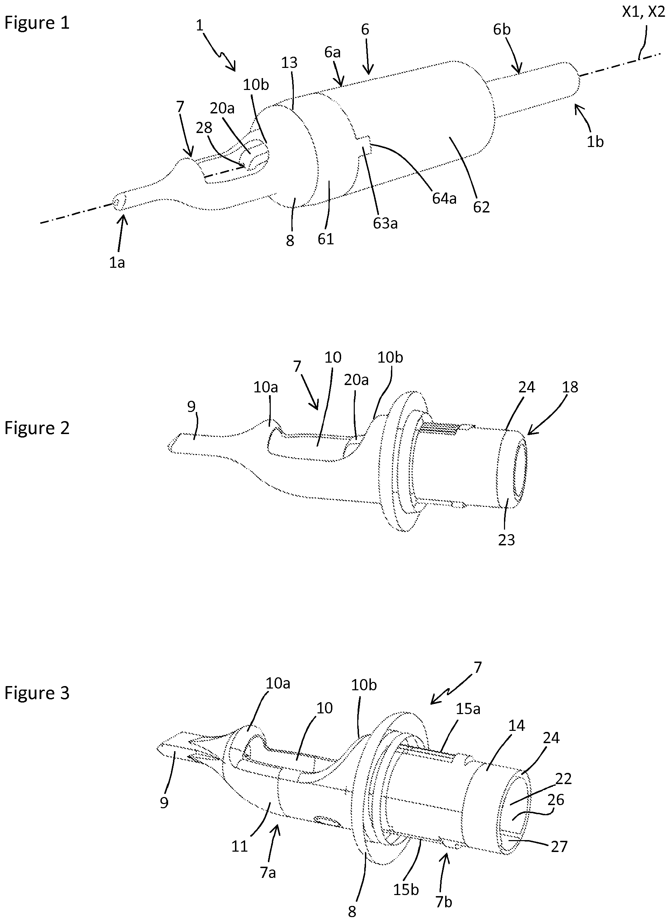

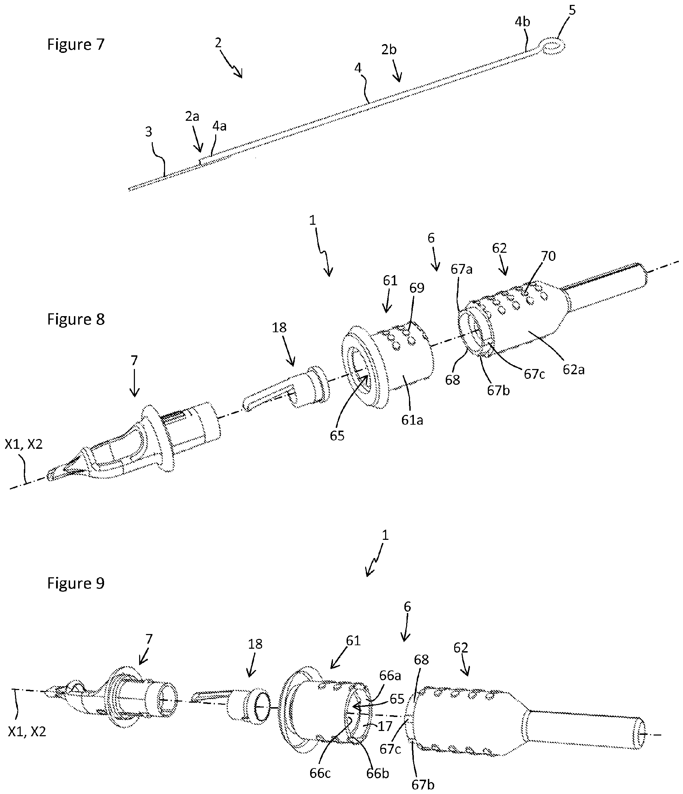

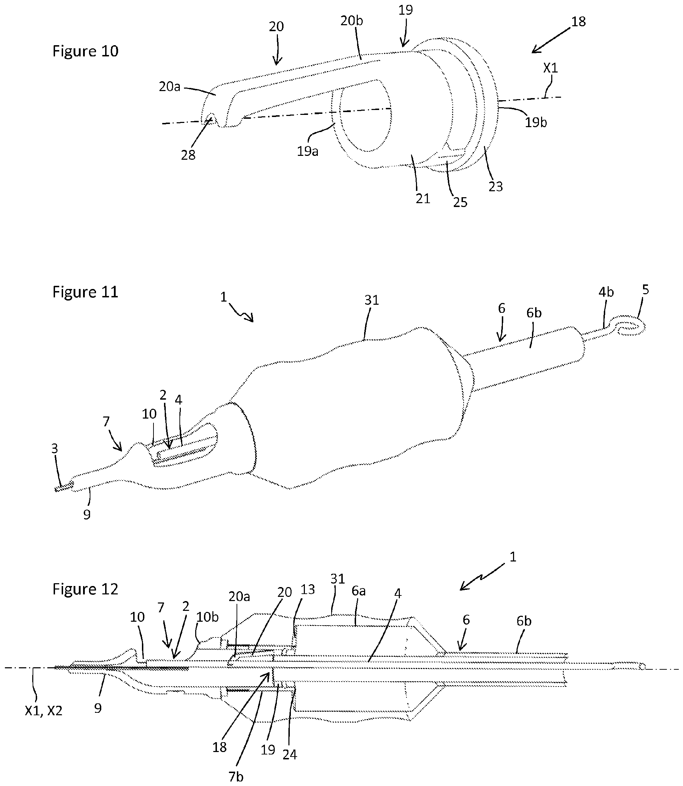

Sur la figure 1 est illustré un tube de tatouage 1 qui sera nommé « tube 1 » dans la suite de la description. Le tube 1 comprend un côté avant 1a et un côté arrière 1b. Un faisceau d'aiguilles 2, illustré en figure 7, traverse de part en part le tube 1 dans le sens de sa longueur. Le faisceau d'aiguilles 2 n'est pas illustré sur la figure 1, mais cela apparaît de manière similaire sur la variante de tube 1 illustrée en figures 11 et 12. Tel qu'illustré en figure 7, le faisceau d'aiguilles 2 comporte dans sa partie avant 2a une seule aiguille 3, l'autre partie 2b dudit faisceau 2 étant constituée d'une tige 4. Le tube 1 permet toutefois la réception de variantes de faisceau d'aiguilles 2 avec plus d'une aiguilles 3 dans la partie avant 2a. L'extrémité avant 4a de la tige 4 est prolongée vers l'avant par la ou les aiguilles 3 et son extrémité arrière 4b comporte une forme d'anneau 5 permettant son assemblage avec un dispositif d'actionnement d'une machine de tatouage (non illustré), une telle machine étant bien connue de l'homme du métier. Traditionnellement, la ou les aiguilles 3 sont fixées par soudure sur la tige 4, l'ensemble étant stérilisé.

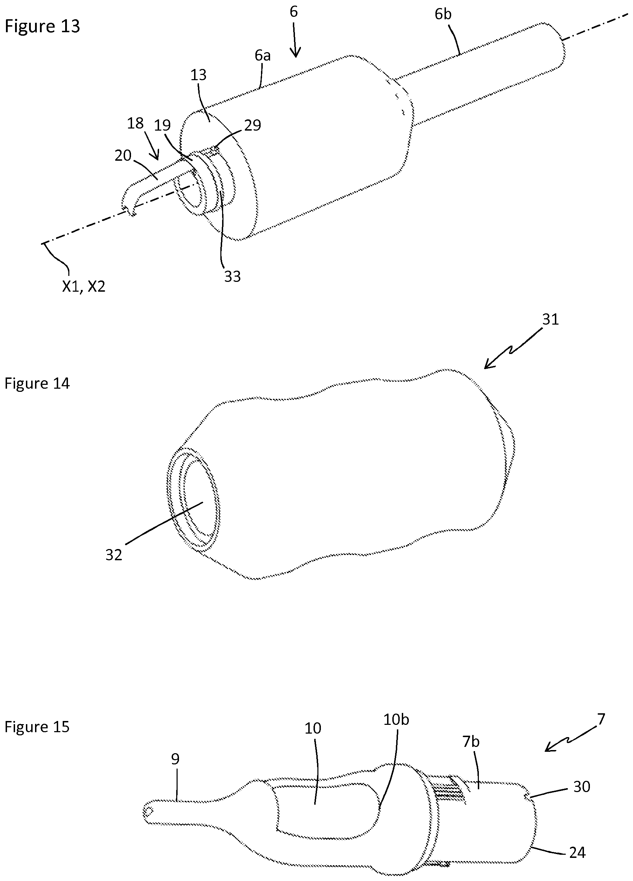

Sur la figure 1, le tube 1 comprend un corps de maintien 6 et un module 7 qui sont assemblés ensemble de manière amovible. Le corps de maintien 6 est creux, de préférence tubulaire, pour permettre le passage du faisceau d'aiguilles 2 et il comprend une partie avant 6a cylindrique qui permet le maintien du tube 1 avec la main et une partie arrière 6b cylindrique dont le diamètre est plus petit que celui de la partie avant 6a, ladite partie arrière 6b permettant la fixation du tube 1 par bridage sur un corps de la machine de tatouage (non illustré). Le corps de maintien 6 est constitué d'une pièce avant 61 et d'une pièce arrière 62 assemblées entre elles grâce à la présence de deux pattes 63a, 63b sur la pièce avant 61 s'engageant dans deux logements 64a, 64b sur la pièce arrière 62, comme l'illustrent les figure 1, 5 et 6. Selon le diamètre du corps de maintien 6, on peut envisager l'ajout d'un manchon complémentaire de forme ergonomique, comparable à celui illustré en figure 14 et mis en oeuvre sur la variante de tube 1 illustrée en figure 11, qui sera détaillée ensuite.

Tel qu'illustré sur la figure 3, le module 7 est également creux pour permettre le passage du faisceau d'aiguilles 2 et il comprend une partie avant 7a et une partie arrière 7b séparées par une collerette 8. La partie avant 7a du module 7 comprend un embout 9 au travers duquel sort l'aiguille 3, comme cela apparaît en figures 11 et 12 sur la variante de tube 1. Cette partie avant 7a comprend une ouverture 10 agencée sur le contour 11 dudit module, ladite ouverture permettant de limiter la réserve d'encre et de la visualiser. Cette ouverture permet également au module 7 d'être à la pression du milieu ambiant, ce qui facilite l'introduction de l'encre par l'embout 9 lors des mouvements de va-et-vient de la ou des aiguilles 3. Le bord avant 10a de l'ouverture 10 est plus ou moins attenant à l'embout 9, comme le montrent les figures 1 et 2. La partie arrière 7b du module 7 est cylindrique et s'emboîte dans un trou 12 cylindrique mis en oeuvre sur la pièce arrière 62 du corps de maintien 6, la collerette 8 venant en butée contre l'extrémité avant 13 dudit corps de maintien 6. La pièce avant 61 comprend également un trou 65 permettant le passage de la partie arrière dans une orientation angulaire définie, afin de venir l'emboîter dans le trou 12 sur la pièce arrière 62. La partie arrière 7b du module 7 est de forme tubulaire et comprend sur son contour externe 14 deux protubérances 15a, 15b qui s'étendent dans le sens de la longueur et qui s'engagent dans deux rainures 16a, 16b respectives agencées sur le contour interne 17 du trou 65. Le contour interne 17 comprend deux portions 17a, 17b séparées par les deux rainures 16a, 16b, ces deux portions 17a, 17b définissant une réduction du diamètre du trou 65, ce qui permet d'introduire la partie arrière 7b dans la pièce avant 61 en positionnant les protubérances 15a, 15b en correspondance avec les rainures 16a, 16b, puis de tourner le module 7 pour venir presser lesdites protubérances 15a, 15b contre les portions 17a, 17b du contour interne 17. Cela permet de bloquer la rotation et la translation du module 7 vis-à-vis du corps de maintien 6 une fois lesdits éléments emboîtés l'un dans l'autre.

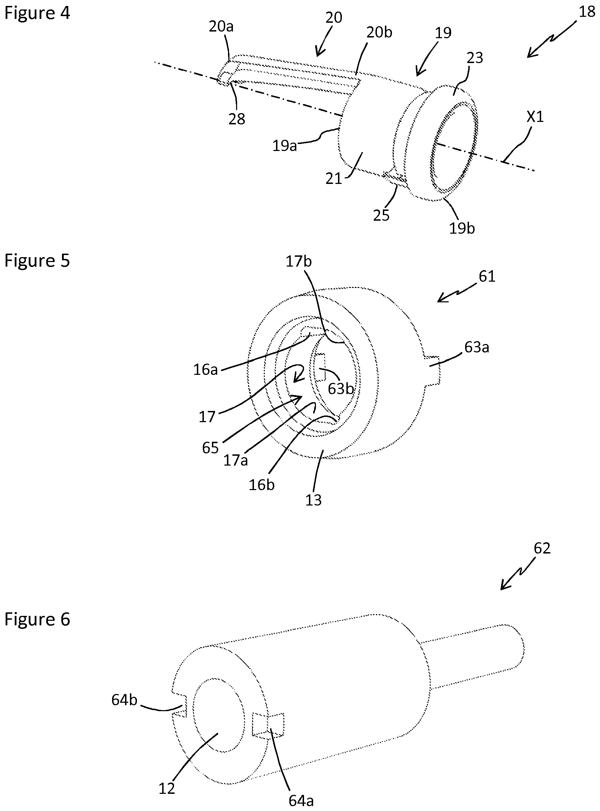

Tel qu'illustré en figure 2, le module 7 reçoit par emboîtement un système de bridage et de guidage 18 du faisceau d'aiguilles 2, appelé « système 18 » dans la suite de la description. Tel qu'illustré en figures 4 et 10, le système 18 comprend un corps 19 qui est cylindrique et un bras 20 qui est fixé au côté avant 19a dudit corps 19 et qui s'étend vers l'avant à l'intérieur dudit module 7, comme le montrent notamment les figures 1 et 2. Le corps 19 est creux pour permettre le passage du faisceau d'aiguilles 2, le contour externe 21 dudit corps 19 s'emboîtant dans un trou 22 (illustré en figure 3) sur la partie arrière 7b du module 7. Le côté arrière 19b du corps 19 comprend bord épaulé 23 qui constitue un élément de butée venant en appui contre l'extrémité arrière 24 du module 7 pour délimiter la position d'arrêt du système 18 sur le module 7. Le contour externe 21 du corps 19 comprend une protubérance 25 qui s'étend en partie dans le sens de la longueur comme le montrent notamment les figures 4 et 10. Le contour interne 26 dudit trou 22 comporte une rainure 27, illustrée en figure 3, qui reçoit ladite protubérance 25 lors de l'emboîtement du corps 19 dans ladite partie arrière 7b, ce qui permet de bloquer la rotation du système 18 vis-à-vis du module 7.

Tel qu'illustré sur les figures 4 et 10, le bras 20 du système 18 à son extrémité avant 20a qui est recourbée vers l'intérieur, cette extrémité avant 20a comprenant une encoche 28 qui présente plus ou moins une forme en V ou en U, dans laquelle vient se loger la tige 4 du faisceau d'aiguilles 2. L'encoche 28 est positionnée plus ou moins dans l'axe X1 de révolution du corps 19, comme l'illustrent notamment les figures 4 et 10, ledit axe X1 étant confondu avec l'axe X2 du tube 4 une fois lesdits éléments assemblés, comme l'illustre notamment la figure 1. L'embout 9, par lequel sortent les aiguilles 3, est également disposé dans cet axe X1. On pourra adapter la forme de l'encoche 28 et sa position vis-à-vis de l'axe X1 en fonction de la forme et de la grosseur de la tige 4.

Tel que l'illustre les figures 1 et 2, lorsque le système 18 est convenablement emboîté dans le module 7, l'extrémité avant 20a du bras 20 est positionnée au niveau du bord arrière 10b de l'ouverture 10 sur ledit module 7 et la courbure de cette extrémité avant 20a est orientée en opposition par rapport à l'ouverture 10. Cette position de l'extrémité avant 20a garantit le maintien convenable de la tige 4 du faisceau d'aiguilles dans l'encoche 28.

Tel qu'illustré en figures 4 et 10, l'extrémité arrière 20b du bras 20 est fixée au côté avant 19a du corps 19, ledit bras 20 s'étendant vers l'avant et étant incliné par rapport à l'axe X1 de sorte que son extrémité avant 20a recourbée munie de l'encoche 28 soit disposée plus ou moins selon cet axe X1. Le système 18 est réalisé dans une matière plastique, ce qui permet au bras 20 de disposer d'une flexibilité de sorte à tolérer une légère déformation de celui-ci pour permettre le passage du faisceau d'aiguilles 2 et pour prendre convenablement appui contre la tige 4 dudit faisceau. Cette flexibilité permet également d'amortir les vibrations lorsque le faisceau d'aiguilles 2 est activé par la machine de tatouage (non illustrée). Dans les cas où la longueur du bras 20 permettrait à son encoche 28 de prendre appui sur la tige 4 à proximité de la soudure permettant la fixation de la ou des aiguilles 3 sur ladite tige 4, cette flexibilité du bras 20 permettrait également de pallier la présence éventuelle de bavures pouvant exister au niveau de ladite soudure, en soulevant l'encoche 28 pour la dégager desdites bavures durant le mouvement de va-et-vient de la tige 4, ce qui éviterait toute saccade durant le tatouage. Toutefois, de préférence, on dimensionnera la longueur du bras 20 pour que son encoche 28 soit hors de portée de l'extrémité avant 4a de la tige 4, durant les mouvements de va-et-vient de cette tige 4.

Le système 18 est emboîté dans la partie arrière 7b du module 7, puis lesdits éléments sont emboîtés dans la partie avant 6a du corps de maintien 6, comme expliqué précédemment, ce qui permet la constitution du tube 1 intégrant ledit système 18. Ainsi, le système 18 est complètement inaccessible une fois en position sur le tube 1. Puis le faisceau d'aiguilles 2 est introduit par le côté arrière 1b du tube 1 jusqu'à ce que la ou les aiguilles 3 sortent par l'embout 9 sur son côté avant 1a. Durant l'introduction du faisceau d'aiguilles 2 dans le tube 1, le bras 20 fléchit légèrement pour permettre à la tige 4 de se loger convenablement dans l'encoche 28. Lors de l'activation de la machine de tatouage (non illustrée), un mouvement de va et vient est transmis au faisceau d'aiguilles 2 dans le sens de la longueur du tube 1, l'encoche 28 assurant un guidage en translation de la tige 4 tout en appuyant légèrement sur celle-ci pour la maintenir en position dans l'axe X2 du tube 1.

Sur les figures 8 et 9 est illustrée une variante du tube 1, la conception dudit tube 1 étant identique à celle décrite précédemment. Seul change l'assemblage entre la pièce avant 61 et de la pièce arrière 62 constituant le corps de maintien 6. La pièce avant 61 comprend trois petites rainures 66a, 66b, 66c agencées sur le contour interne 17 du trou 65 et la pièce arrière 62 comprend trois petits bourrelets 67a, 67b, 67c agencés sur une portion cylindrique 68. Les trois petits bourrelets 67a, 67b, 67c viennent se loger respectivement dans lesdites petites rainures 66a, 66b, 66c lors de l'emboîtement de la portion cylindrique 68 dans le trou 65. La répartition non uniforme des petites rainures 66a, 66b, 66c autour du contour interne 17 et des petits bourrelets 67a, 67b, 67c autour de la portion cylindrique 68 assure un positionnement précis de la pièce avant 61 sur la pièce arrière 62. Tel qu'illustré sur ces figures 8 et 9, les pièces avant 61 et arrière 62 comprennent des crampons 69, 70 sur leurs contours externes 61a, 62a respectifs, lesdits crampons 69, 70 étant alignés du fait du positionnement précis lors de l'emboîtement desdites pièces 61, 62. Ces crampons 69, 70 assurent un meilleur grippage d'un manchon (non illustré mais comparable au manchon 31 sur les figures 11 et 14) disposé autour du corps de maintien 6 afin de disposer d'une meilleure préhension du tube 1 durant une opération de tatouage.

Sur les figures 11 à 15 est illustrée une autre variante du tube 1. Le tube 1 comprend toujours un module 7, un corps de maintien 6 et un système 18. A la différence des variantes précédentes, le corps de maintien 6 est constitué d'une seule pièce et, en outre, ledit corps de maintien 6 et le système 18 forment une seule et même pièce, le corps 19 dudit système 18 étant fixé à l'extrémité avant 13 du corps de maintien 6 qu'il prolonge, comme cela apparaît sur la figure 13.

Tel qu'illustré en figure 12, la partie arrière 7b du module 7 s'emboîte sur le corps 19 du système 18 et a son extrémité arrière 24 qui vient en appui contre l'extrémité avant 13 du corps de maintien 6, ce qui délimite la position du système 18 à l'intérieur du module 7. Le bras 20 du système 18 s'étend vers l'avant dans la partie arrière 7b du module 7, son extrémité recourbée 20a demeurant toutefois en amont du bord arrière 10b de l'ouverture 10 sur ledit module 7.

Tel qu'illustré en regard des figures 12, 13 et 15, le corps 19 du système 18 comprend au niveau de son extrémité arrière 19b un plot 29 et le module 7 comprend à son extrémité arrière 24 une encoche 30. L'encoche 30 vient se loger sur le plot 29 lors de l'emboîtement du module 7 sur le corps 19 du système 18. Cela permet de définir une position précise du système 18 sur le module 7 et bloque la rotation entre ces deux pièces.

Tel qu'illustré sur les figures 11, 12 et 14, le tube 1 comprend un manchon 31 dans un matériau souple, par exemple de l'élastomère qui se positionne autour de la partie avant 6a du corps de maintien 6 et autour de la partie arrière 7b du module 7. Le manchon 31 comprend dans sa partie avant 31a une ouverture 32 qui permet d'abord d'engager le corps de maintien 6 et le système 18 à l'intérieur de celui-ci, la partie arrière 7b du module s'emboîtant ensuite dans cette ouverture 32 puis sur le corps 19 du système 18 en logeant le plot 29 dans l'encoche 30. La pénétration du corps de maintien 6 dans le manchon 31 est possible grâce à la déformation de la matière dudit manchon 31. Ce manchon 31 dispose d'une forme ergonomique pour une bonne prise en main du tube 1.

Tel qu'illustré sur la figure 13, le corps 19 du système 18 comprend un bourrelet circulaire 33 qui assure en montage serré de la partie arrière 7b du module 7 sur ledit corps 19, de sorte à bloquer la translation du module 7 selon l'axe X1 du tube 1.

De nombreuses variantes du tube 1 pourront être envisagées dans le cadre de l'invention, le corps de maintien 6 et le système 18 pouvant être deux pièces individuelles ou une seule et même pièce, comme décrit précédemment. On peut également prévoir de modifier la position d'arrêt de l'encoche 28 du bras 20, disposée en amont du bord arrière 10b de l'ouverture 10 sur le module 7. On peut également adapter un manchon 31 sur des variantes de tubes 1 de conceptions comparables à celles des figures 1 à 10 pour les deux premières variantes, de sorte à permettre une meilleure préhension desdits tubes 1.

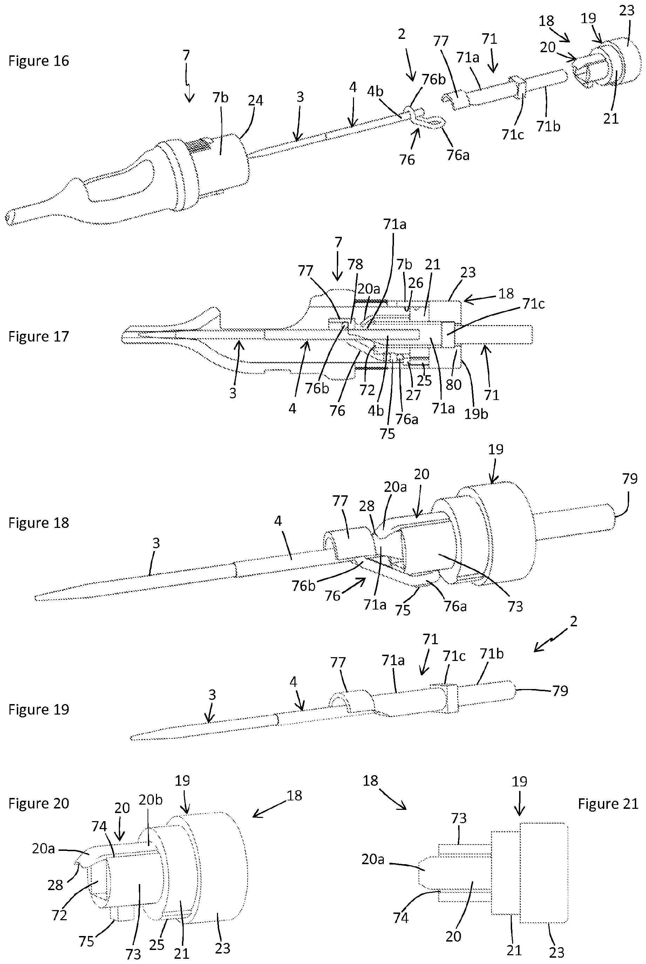

Dans une autre variante illustrée en regard des figures 16 à 21, le module 7 intègre un faisceau d'aiguille 2 et un système 18 dont les conceptions diffèrent par rapport à celles décrites précédemment en regard des figures 1 à 15. Ce type de module 7 est à usage unique. Le module 7 en tant que tel comporte une conception similaire à celles décrites précédemment pour les variantes des figures 1 à 15 ; on pourra donc se référer à ces figures 1 à 15 pour la conception du module 7 selon cette nouvelle variante des figures 16 à 21.

Selon cette mise en oeuvre des figures 16 à 21, le contour externe 21 du corps 19 du système 18 est emboîté comme précédemment sur la partie arrière 7b du module 7 jusqu'à ce que le bord épaulé 23 du corps 19 vienne en butée contre l'extrémité arrière 24 du module. Le contour externe 21 du corps 19 comprend une protubérance 25 qui vient se loger dans une rainure 27 sur le contour interne 26 du module 7, comme l'illustrent les figures 17 et 20, de sorte à bloquer la rotation du système 18 vis-à-vis du module 7.

Le faisceau d'aiguille 2 comporte une ou plusieurs aiguilles 3 prolongées par une tige 4 qui est courte, l'extrémité arrière 4b de la tige 4 étant fixée dans la partie avant 71a d'un coulisseau 71 par collage ou par emboîtement serré. Le coulisseau 71 est monté en glissière dans le corps 19 du système 18, sa partie arrière 71b dépassant de l'extrémité arrière 19b du corps 19. Le montage en liaison glissière est réalisé au moyen d'un trou carré 72 agencé sur le corps 19, qui reçoit et guide une portion carrée 71c sur le coulisseau 7. Le corps 19 comprend une extension 73 qui prolonge vers l'avant le contour externe 21 et qui comprend une fente 74 dans laquelle est logé le bras 20, comme l'illustrent les figures 20 et 21. Ainsi, le bras 20 conserve toute sa flexibilité vis-à-vis du corps 19. L'encoche 28 à l'extrémité recourbée 20a du bras 20 prend appui sur la partie avant 71a du coulisseau 71, comme l'illustrent les figures 17 et 18.

Cette extension 73 comprend un plot 75 qui reçoit une partie arrière 76a d'un élastique 76, comme l'illustrent les figures 17 et 18. L'extrémité avant du coulisseau 71 comporte un élément en demi-lune 77 dans lequel vient se loger la partie avant 76b de cet élastique 76, la tige 4 passant au travers de cette partie avant 76b de l'élastique 76, comme l'illustrent les figures 16 à 19. La partie avant 76b vient en butée contre un épaulement 78 dans le fond de l'élément en demi-lune 77, comme le montre la figure 17. Lors d'un mouvement vers l'avant du faisceau d'aiguilles 2, l'élastique 76 se tend du fait que sa partie arrière 76a et sa partie avant 76b sont respectivement maintenues par le plot 75 et par l'épaulement 78. Ce mouvement vers l'avant du faisceau d'aiguilles 2 est assuré par la machine de tatouage qui comprend une tige complémentaire (non illustrée) actionnée en va-et-vient par un dispositif d'actionnement, cette tige complémentaire pénétrant dans le corps de maintien 6 du tube 1pour venir pousser l'extrémité arrière 79 du coulisseau 71 qui est logée dans ledit corps de maintien 6 du tube 1 une fois le module 7 avec le système 18 engagés sur ledit corps de maintien 6. Ce corps de maintien 6 est par exemple de conception semblable à l'une des variantes décrites précédemment. Lorsque ladite tige complémentaire est remontée par le dispositif d'actionnement et qu'elle se dégage de l'extrémité arrière 79 du coulisseau 71, l'élastique 76 assure le rappel en arrière du faisceau d'aiguille 2, jusqu'à ce que le portion carrée 71 du coulisseau vienne en butée contre un second épaulement 80 agencé dans le fond du trou carré 72, comme le montre la figure 17.

La description détaillée qui précède de plusieurs modes de réalisation de l'invention n'a aucun caractère limitatif. Bien au contraire, elle a pour objectif d'ôter toute éventuelle imprécision quant à sa portée.

L'invention trouvera son application chez les tatoueurs et sera mis en oeuvre par des plasturgistes qui fabriquent des produits consommables pour le tatouage.

Selon les différentes variantes de conceptions, le système 18 pourra être vendu prémonté avec le module 7 pour venir s'emboîter dans un corps de maintien 6 et recevoir un faisceau d'aiguilles 2, dans le cas des réalisations des figures 1 à 9 ou de toutes autres variantes s'apparentant. Le système 18 pourra être vendu prémonté avec le module 7, le faisceau d'aiguilles 2 et l'élastique 76 dans le cas de réalisation des figures 16 à 21 ou de toutes autres variantes s'apparentant.

## Revendications

1. Système de bridage et de guidage (18) d'un faisceau d'aiguilles (2) de tatouage, **caractérisé en ce qu'**il comprend un corps (19) creux configuré pour recevoir une partie (4, 71) du faisceau d'aiguilles (2) et pour être emboîté dans une position définie à l'intérieur d'un module (7) d'un tube de tatouage (1) et un bras (20) qui s'étend au-delà d'un côté avant du corps (19) auquel il est fixé, le bras (20) comprenant une extrémité (20a) recourbée munie d'une encoche (28) configurée pour recevoir ladite partie (4, 71) du faisceau d'aiguilles (2), ledit bras (20) étant configuré pour que l'encoche (28) appuie sur ladite partie (4, 71).

2. Système de bridage et de guidage (18) selon la revendication 1, dans lequel le corps (19) est configuré pour être emboîté à l'intérieur d'un corps de maintien (6) du tube de tatouage (1).

3. Système de bridage et de guidage (18) selon la revendication 1, dans lequel le corps (19) constitue un prolongement avant d'un corps de maintien (6) du tube de tatouage (1) auquel ledit corps (19) est intégré.

4. Système de bridage et de guidage (18) selon l'une des revendications précédentes, dans lequel le corps (19) comprend un axe de révolution (X1), l'encoche (28) étant positionnée sensiblement dans ledit axe (X1) pour prendre appui sur une tige (4) du faisceau d'aiguille (2).

5. Système de bridage et de guidage (18) selon la revendication 4, dans lequel le bras (20) est incliné par rapport à l'axe de révolution (X1) pour que son extrémité recourbée (20a) soit rapprochée dudit axe (X1).

6. Système de bridage et de guidage (18) selon l'une des revendication 1 ou 2, dans lequel le corps (19) comprend un trou (72) configuré pour guider en translation un coulisseau (71) du faisceau d'aiguilles (2).

7. Système de bridage et de guidage (18) selon la revendication 6, dans lequel le bras (20) est configuré pour que son encoche (28) prenne appui sur une partie avant (71a) du coulisseau (71).

8. Système de bridage et de guidage (18) selon l'une des revendications 6 ou 7, dans lequel le corps (19) comprend une extension (73) qui prolonge vers l'avant ledit corps, l'extension (73) comprenant un plot (75) pour le maintien d'un élastique (76) de rappel du faisceau d'aiguilles (2) et une fente (74) dans laquelle est logé le bras (20).

9. Système de bridage et de guidage (18) selon l'une des revendications précédentes, dans lequel le bras (20) est flexible vis-à-vis du corps (19).

10. Système de bridage et de guidage (18) selon l'une des revendications précédentes, dans lequel le corps (19) comprend un élément de butée (23) configuré pour arrêter la position dudit système (18) dans le sens de la longueur du module (7) du tube de tatouage (1).

11. Système de bridage et de guidage (18) selon l'une des revendications précédentes, dans lequel le corps (19) comprend un élément de blocage (25, 29) configuré pour bloquer la rotation dudit système vis-à-vis du module (7) du tube de tatouage (1).

12. Tube de tatouage (1) comprenant un module (7), un corps de maintien (6) et un système de bridage et de guidage (18) selon l'une des revendications précédentes, le corps (19) du système de bridage et de guidage (18) étant emboîté dans une partie arrière (7b) du module (7), le module (7) étant monté de manière amovible vis-à-vis du corps de maintien (6) et du système de bridage et de guidage (18).

13. Tube de tatouage (1) selon la revendication 12, dans lequel la partie arrière (7b) du module (7) comprend un élément de blocage (27, 30) recevant un élément de blocage (25, 29) complémentaire du corps (19) du système de bridage et de guidage (18) lors de l'emboîtement dudit corps (19) dans ladite partie arrière (7b), de sorte à bloquer la rotation entre lesdits éléments.

14. Tube de tatouage (1) selon l'une des revendications 12 ou 13, dans lequel le module (7) comprend un contour (11) muni d'une ouverture (10), le système de bridage et de guidage (18) ayant son encoche (28) positionnée en amont de l'ouverture (10).

15. Tube de tatouage (1) selon l'une des revendications 12 à 14, lequel comprend des moyens de préhension facilitant sa manipulation.

## Patentansprüche

1. System zum Einspannen und Führen (18) eines Tätowiernadelstrangs (2), **dadurch gekennzeichnet, dass** es einen hohlen Körper (19), der so konfiguriert ist, dass er einen Teil (4, 71) des Nadelstrangs (2) aufnimmt und in einer definierten Position im Inneren eines Moduls (7) eines Tätowierrohrs (1) eingesetzt werden kann, und einen Arm (20) umfasst, der sich über eine Vorderseite des Körpers (19), an der er befestigt ist, hinaus erstreckt, wobei der Arm (20) ein gebogenes Ende (20a) umfasst, das mit einer Kerbe (28) ausgestattet ist, die so konfiguriert ist, dass sie den Teil (4, 71) des Nadelstrangs (2) aufnimmt, wobei der Arm (20) so konfiguriert ist, dass die Kerbe (28) auf dem Teil (4, 71) aufliegt.

2. Einspann- und Führungssystem (18) nach Anspruch 1, wobei der Körper (19) so konfiguriert ist, dass er in das Innere eines Haltekörpers (6) des Tätowierrohrs (1) eingesetzt werden kann.

3. Einspann- und Führungssystem (18) nach Anspruch 1, wobei der Körper (19) eine vordere Verlängerung eines Haltekörpers (6) des Tätowierrohrs (1), in den der Körper (19) eingebaut wird, darstellt.

4. Einspann- und Führungssystem (18) nach einem der vorstehenden Ansprüche, wobei der Körper (19) eine Rotationsachse (X1) umfasst, wobei die Kerbe (28) im Wesentlichen so in der Achse (X1) positioniert ist, dass sie auf einem Schaft (4) des Nadelstrangs (2) in Auflage geht.

5. Einspann- und Führungssystem (18) nach Anspruch 4, wobei der Arm (20) in Bezug auf die Rotationsachse (X1) so geneigt ist, dass sein gebogenes Ende (20a) der Achse (X1) angenähert ist.

6. Einspann- und Führungssystem (18) nach einem der Ansprüche 1 oder 2, wobei der Körper (19) ein Loch (72) umfasst, das so konfiguriert ist, dass es einen Schieber (71) des Nadelstrangs (2) in der Verschiebung führt.

7. Einspann- und Führungssystem (18) nach Anspruch 6, wobei der Arm (20) so konfiguriert ist, dass seine Kerbe (28) auf einem vorderen Teil (71a) des Schiebers (71) in Auflage geht.

8. Einspann- und Führungssystem (18) nach einem der Ansprüche 6 oder 7, wobei der Körper (19) eine Erstreckung (73) umfasst, die den Körper nach vorne verlängert, wobei die Erstreckung (73) einen Klotz (75) zum Halten eines Gummibandes (76) zum Rückstellen des Nadelstrangs (2) und einen Schlitz (74) umfasst, in dem der Arm (20) aufgenommen ist.

9. Einspann- und Führungssystem (18) nach einem der vorstehenden Ansprüche, wobei der Arm (20) dem Körper (19) gegenüber flexibel ist.

10. Einspann- und Führungssystem (18) nach einem der vorstehenden Ansprüche, wobei der Körper (19) ein Anschlagelement (23) umfasst, das so konfiguriert ist, dass es die Position des Systems (18) in Richtung der Länge des Moduls (7) des Tätowierrohrs (1) stoppt.

11. Einspann- und Führungssystem (18) nach einem der vorstehenden Ansprüche, wobei der Körper (19) ein Sperrelement (25, 29) umfasst, das so konfiguriert ist, dass es die Drehung des Systems dem Modul (7) des Tätowierrohrs (1) gegenüber sperrt.

12. Tätowierrohr (1), das ein Modul (7), einen Haltekörper (6) und ein Einspann- und Führungssystem (18) nach einem der vorstehenden Ansprüche umfasst, wobei der Körper (19) des Einspann- und Führungssystems (18) in einen hinteren Teil (7b) des Moduls (7) eingesetzt ist, wobei das Modul (7) dem Haltekörper (6) und dem Einspann- und Führungssystem (18) gegenüber abnehmbar angebracht ist.

13. Tätowierrohr (1) nach Anspruch 12, wobei der hintere Teil (7b) des Moduls (7) ein Sperrelement (27, 30) umfasst, das beim Einsetzen des Körpers (19) in den hinteren Teil (7b) ein komplementäres Sperrelement (25, 29) des Körpers (19) des Einspann- und Führungssystems (18) aufnimmt, um die Drehung zwischen den Elementen zu sperren.

14. Tätowierrohr (1) nach einem der Ansprüche 12 oder 13, wobei das Modul (7) eine Kontur (11) umfasst, die mit einer Öffnung (10) ausgestattet ist, wobei die Kerbe (28) des Einspann- und Führungssystems (18) stromaufwärts von der Öffnung (10) positioniert ist.

15. Tätowierrohr (1) nach einem der Ansprüche 12 bis 14, das Greifmittel umfasst, die seine Handhabung erleichtern.

## Claims

1. A system for clamping and guiding (18) a bundle of tattoo needles (2), **characterised in that** it comprises a hollow body (19) configured to receive a section (4, 71) of the bundle of needles (2) and to be nested into a defined position within a module (7) of a tattoo tube (1) and an arm (20) which extends beyond a front side of the body (19) to which it is fixed, the arm (20) comprising a curved end (20a) provided with a notch (28) configured to receive said section (4, 71) of the bundle of needles (2), said arm (20) being configured so that the notch (28) bears on said section (4, 71).

2. The clamping and guiding system (18) according to claim 1, wherein the body (19) is configured to be nested within a body (6) for holding the tattoo tube (1).

3. The clamping and guiding system (18) according to claim 1, wherein the body (19) constitutes a front extension of a body (6) for holding the tattoo tube (1) into which said body (19) is integrated.

4. The clamping and guiding system (18) according to one of the preceding claims, wherein the body (19) comprises an axis of revolution (X1), the notch (28) being positioned substantially in said axis (X1) to bear on a rod (4) of the needle bundle (2).

5. The clamping and guiding system (18) according to claim 4, wherein the arm (20) is inclined relative to the axis of revolution (X1) so that its curved end (20a) is brought closer to said axis (X1).

6. The clamping and guiding system (18) according to one of claims 1 or 2, wherein the body (19) comprises a hole (72) configured to guide a slide (71) of the needle bundle (2) in translation.

7. The clamping and guiding system (18) according to claim 6, wherein the arm (20) is configured so that its notch (28) bears on a front section (71a) of the slide (71).

8. The clamping and guiding system (18) according to one of claims 6 or 7, wherein the body (19) comprises an extension (73) which extends said body forward, the extension (73) comprising a stud (75) for maintaining an elastic band (76) for returning the bundle of needles (2) and a slot (74) wherein the arm (20) is housed.

9. The clamping and guiding system (18) according to one of the preceding claims, wherein the arm (20) is flexible with respect to the body (19).

10. The clamping and guiding system (18) according to one of the preceding claims, wherein the body (19) comprises a stop element (23) configured to stop the position of said system (18) in the direction of the length of the module (7) of the tattoo tube (1).

11. The clamping and guiding system (18) according to one of the preceding claims, wherein the body (19) comprises a blocking element (25, 29) configured to block the rotation of said system with respect to the module (7) of the tattoo tube (1).

12. A tattoo tube (1) comprising a module (7), a holding body (6) and a clamping and guiding system (18) according to one of the preceding claims, the body (19) of the clamping and guiding system (18) being nested into a rear section (7b) of the module (7), the module (7) being removably mounted relative to the holding body (6) and the clamping and guiding system (18).

13. The tattoo tube (1) according to claim 12, wherein the rear section (7b) of the module (7) comprises a blocking element (27, 30) receiving a blocking element (25, 29) complementary to the body (19) of the clamping and guiding system (18) during the nesting of said body (19) into said rear section (7b), so as to block the rotation between said elements.

14. The tattoo tube (1) according to one of claims 12 or 13, wherein the module (7) comprises a contour (11) provided with an opening (10), the clamping and guiding system (18) having its notch (28) positioned upstream of the opening (10).

15. The tattoo tube (1) according to one of claims 12 to 14, which comprises gripping means facilitating its handling.
